# NEUE EUROPÄISCHE PATENTSCHRIFT

(11) **EP 0 431 327 B2**
(45) Veröffentlichungstag und Bekanntmachung des Hinweises auf die Entscheidung über den Einspruch: **04.04.2001**
(45) Hinweis auf die Patenterteilung: 24.04.1996
(21) Anmeldenummer: 90121189.6
(22) Anmeldetag: 06.11.1990
(51) Int. Cl.: A61K 39/145, A61K 39/385

(54) **Synthetische Vakzine zur spezifischen Induktion zytotoxischer T-Lymphozyten**
Synthetic vaccine for the specific induction of cytotoxic T-lymphocytes
Vaccin synthétique pour l'induction spécifique des lymphocytes-T-cytotoxiques

(30) Priorität: 10.11.1989 DE 3937412
(43) Veröffentlichungstag der Anmeldung: 12.06.1991
(73) Patentinhaber: Aventis Pharma Deutschland GmbH, 65929 Frankfurt am Main (DE)
(72) Erfinder: Jung, Günther, W-7400 Tübingen (DE); Rammensee, Hans-Georg, W-7400 Tübingen (DE); Deres, Karl, W-7400 Tübingen (DE); Wiesmüller, Karl-Heinz, W-7400 Tübingen (DE)

(56) Entgegenhaltungen:
- EP-A- 0 000 300
- EP-A- 0 203 676
- EP-A- 0 210 412
- EP-A- 0 338 437
- WO-A-89/02277
- WO-A-89/07448
- Nature (1991), vol.351,pages 290 to 296, K.Falk et al.;"Allele specific motifs revealed by sequencing of self-peptides eleuted from MHC molecules".
- Nature. Band 342.S. 561-564. Deres et al.
- EMBO Journal, Band 7, 1988.S. 93-100.Rothbard et al.
- Cell Band 52, 1988.S. 253-258. Bodmer et al.

## Beschreibung

Die vorliegende Erfindung betrifft eine synthetische Vakzine zur spezifischen Induktion zytotoxischer T-Lymphozyten.

Zytotoxische T-Lymphozyten (Killer-T-Zellen) stellen einen wesentlichen Teil der Immunantwort von Warmblütern gegen intrazelluläre Infektionen dar. Zytotoxische T-Lymphozyten werden normalerweise nur mittels einer in-vivo-Impfung mit infektiösen Erregern induziert (J. Bastin et al., J. Exp. Med., Vol 165, June 1987). Wegen der damit verbundenen Risiken würde ein synthetischer Impfstoff für die spezifische Induktion von zytotoxischen T-Lymphozyten eine erhebliche Verbesserung darstellen. Es wurde nun überraschenderweise gefunden, daß durch die Verwendung bestimmter Membranankerwirkstoffkonjugate enthaltend Killer-T-Zellepitope die spezifische in-vivo-Induktion von zytotoxischen T-Lymphozyten möglich ist.

Es ist zwar bereits bekannt, daß Membranankerwirkstoffkonjugate zur Erzeugung neutralisierender Antikörper geeignet sind (vgl. Angew. Chem. 97 (1985), Nr. 10, S. 883 ff.); über eine synthetische Vakzine enthaltend Membranankerwirkstoffkonjugate zur spezifischen Induktion von zytotoxischen T-Lymphozyten wurde jedoch bisher nicht berichtet.

Erfindungsgegenstand ist demzufolge eine synthetische Vakzine zur Induktion von zytotoxischen T-Lymphozyten, bestehend aus einem Konjugat aus mindestens einer Membranankerverbindung und einem mindestens ein Killer-T-Zellepitop enthaltenden Protein eines Virus, eines Bakteriums, eines Parasiten oder eines Tumorantigens oder mindestens einer Partialsequenz enthaltend mindestens ein Killer-T-Zellepitop eines Virus-, Bakterium- oder Parasiten-Proteins oder eines Tumorantigens wobei die Partiasequenzen des Maul und Klauenseuche Virus ausgenommen sind, dadurch gekennzeichnet, daß die Membranankerverbindung eine der nachstehenden Formeln aufweist 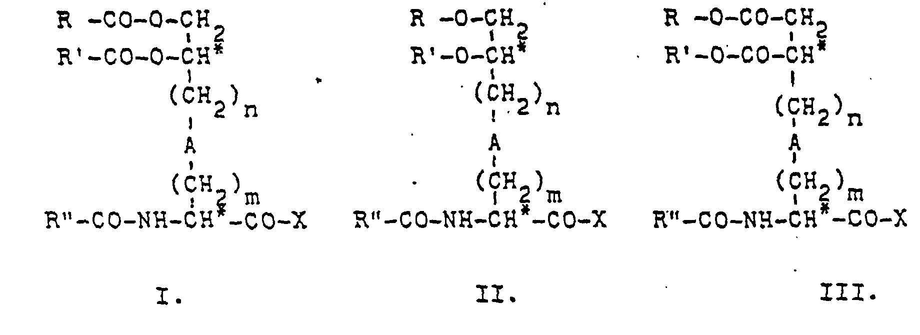 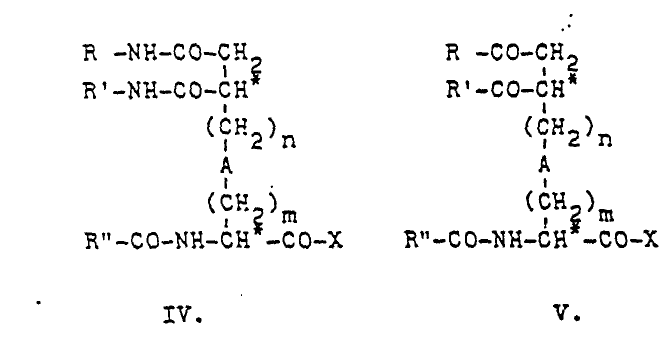 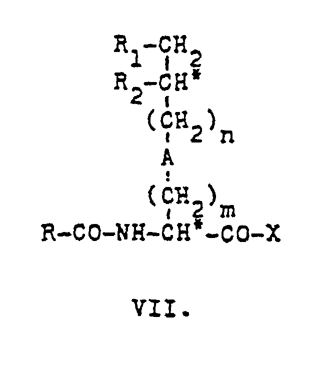 in denen
A Schwefel, Sauerstoff, Disulfid (-S-S-), Methylen (-CH₂-) oder -NH- sein kann;
n = 0 bis 5, m = 1 oder 2 ;
C^{*} ein asymmetrisches Kohlenstoffatom mit R- oder S-Konfiguration ist,
R, R' und R" gleich oder verschieden sind und Wasserstoff oder eine Alkyl-, Alkenyl- oder Alkinylgruppe mit 7 bis 25 Kohlenstoffatomen sind, welche mit Hydroxy-, Amino-, Oxo-, Acyl-, Alkyl- oder Cycloalkylgruppen substituiert sein kann, und R₁ und R₂ gleich oder verschieden sind und dieselben Bedeutungen wie R, R' und R" haben, aber auch -OR, -O-COR, -COOR, -NHCOR oder -CONHR sein können, wobei X eine Kette von bis zu 10 Aminosäuren ist, an die das Protein oder die Partialsequenz des Virus-, Bakterium- oder Parasiten-Proteins oder eines Tumorantigens gebunden ist, oder das Protein oder die Partialsequenz selbst ist.

Von diesen sind als Beispiele besonders hervorzuheben: In bakteriellem Lipoprotein vorkommende N-Termini, wie z.B.: Y-Ser-Ser-Ser-Asn, Y-Ile-Leu-Leu-Ala, Y-Ala-Asn-Asn-Gln, Y-Asn-Ser-Asn-Ser, Y-Gly-Ala-Met-Ser, Y-Gln-Ala-Asn-Tyr, Y-Gln-Val-Asn-Asn, Y-Asp-Asn-Ser-Ser, wobei Y einer der unter Formel I bis V aufgeführten Reste sein kann. Diese Lipopentapeptide können auch in verkürzter Form (Lipodi-, Lipotri- oder Lipotetrapeptide) als Membranankerverbindung eingesetzt werden. Ganz besonders bevorzugt ist N-Palmitoyl-S-[2,3(bispalmitoyloxy)propyl]-cysteinyl-seryl-serin (Pam₃Cys-Ser-Ser), N-Palmitoyl-S-[2,3-(bispalmitoyloxy)-propyl]-cysteinyl-seryl-glycin und N-Palmitoyl-S-(2,3-(bis-palmitoyloxy)propyl]-cysteinyl-alanyl-D-isoglutamin. Weiterhin besonders bevorzugt sind die Verbindungen der Formeln I und III, insbesondere Verbindungen der Formel I.

Der Substituent A ist vorzugsweise Schwefel oder Methylen, besonders bevorzugt Schwefel.

Die Substituenten R, R' und R" sind vorzugsweise Alkylreste mit 14 bis 18 C-Atomen; besonders bevorzugt sind Alkylreste mit 16 C-Atomen.

Der Substituent X wird vorzugsweise gebildet aus 1 bis 2 polaren Aminosäureresten, besonders bevorzugt ist der Serin-Rest.

Für die erfindungsgemäße Vakzine eignen sich zur Kopplung an die Membranankerverbindung unterschiedliche Proteine oder Protein-Partialsequenzen von intrazellulär auftretenden Krankheitserregern, Viren-, Bakterien- oder Parasiten-Proteinen oder von Tumor-Antigenen, die von Killer-T-Zellen erkannt werden.

Solche Proteine oder Partialsequenzen (auch als Killer-T-Zell-Epitope bezeichnet) zeichnen sich dadurch aus, daß sie von zytotoxischen T-Lymphozyten zusammen mit MHC-Molekülen (major histocompatibility complex) erkannt werden.

Die erfindungsgemäße Vakzine eignet sich zur Immunisierung gegen alle Erreger, die Killer-T-Zell-Epitope aufweisen, wie z. B. gegen Adenoviren, HIV, Influenza-Viren, LCMV, MCMV, Hepatitis-Viren, HTLV, FELV, Treponema pallidum, Gonokokkus, Bordetella pertussis, Plasmodien, Listerien, Mycobakterien oder Leishmanien. Bisher bereits bekannte Killer-T-Zell-Epitope sind die in der nachfolgenden Tabelle aufgeführten Partialsequenzen, von denen das Influenza-Nukleoprotein P₃CSS-NP 147-158 (R⁻) und die HIV-Epitope eine Sonderstellung einnehmen.

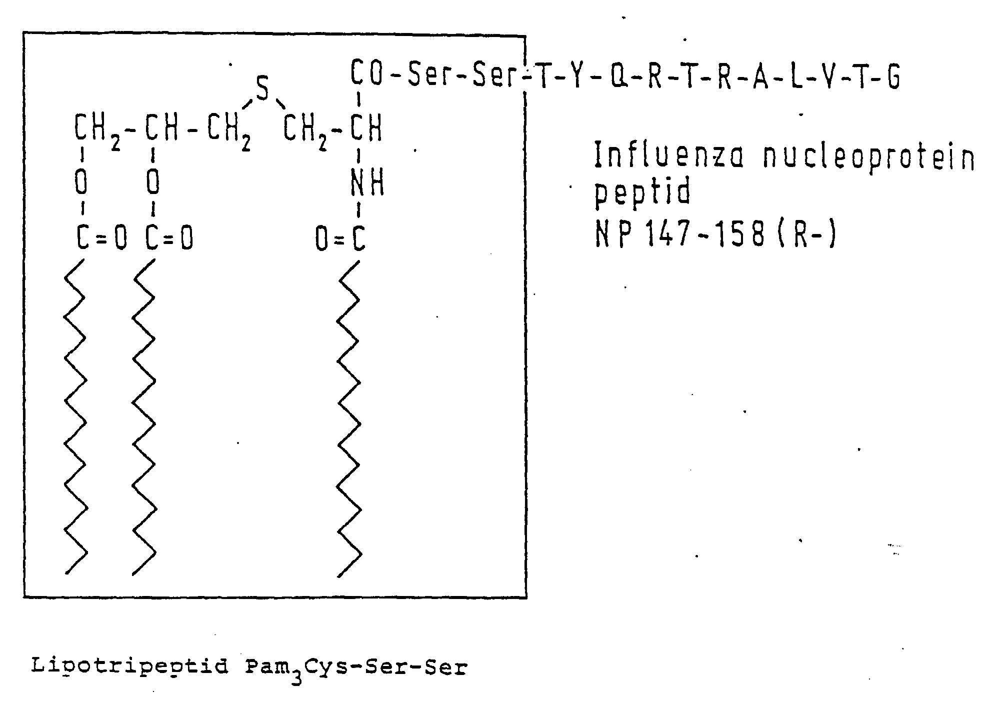

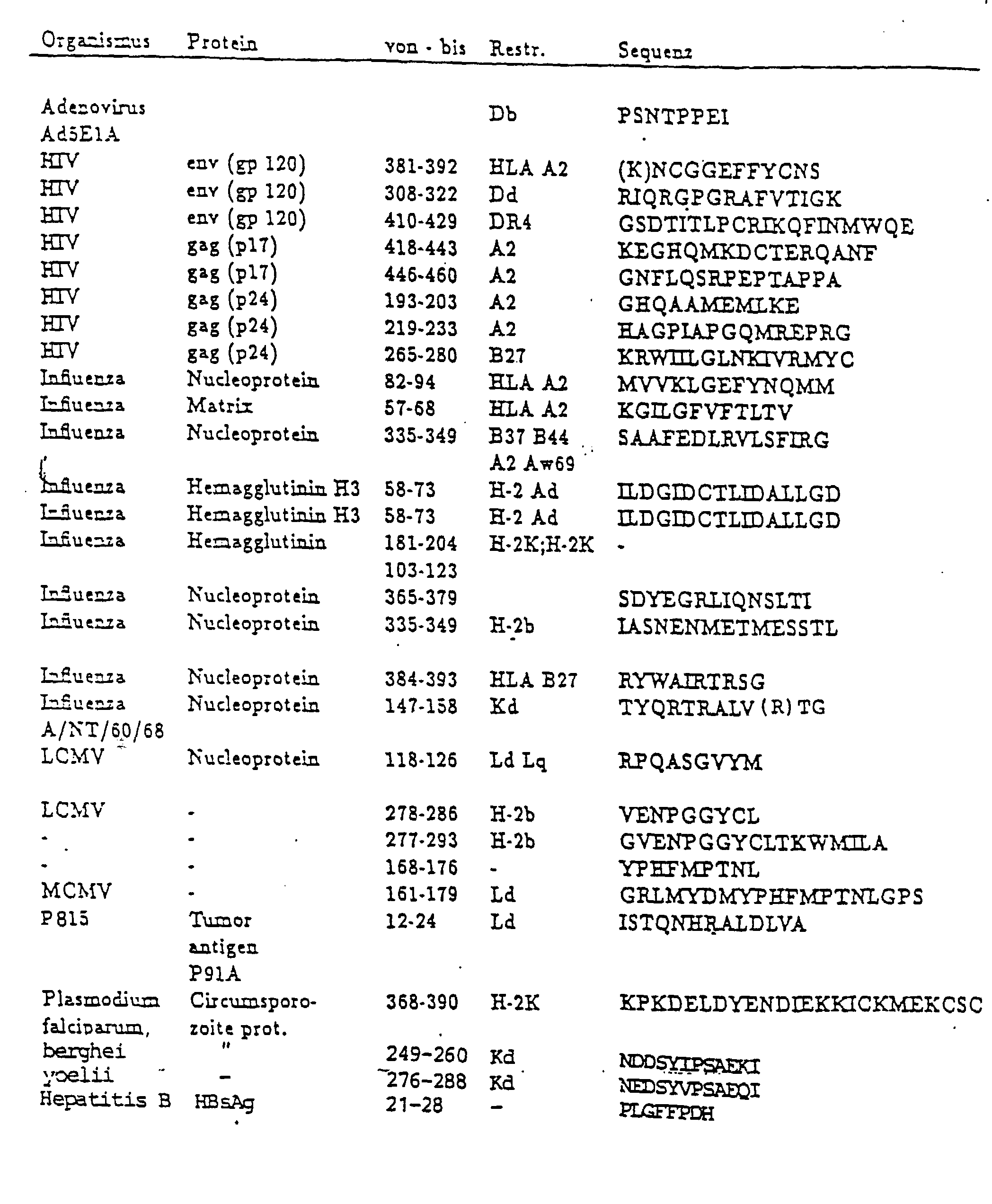

Mit Hilfe der erfindungsgemäßen Vakzine ist es darüber hinaus möglich, verschiedene Membranankerverbindungen, gekoppelt an verschiedene Partialsequenzen zu mischen, um eine auf ein bestimmtes Ziel optimal abgestimmte Vakzine zu erhalten. Weiterhin kann eine entsprechende Mischung zusätzlich Membranankerwirkstoffkonjugate enthalten, die die humorale Immunantwort stimulieren und zusätzlich zur Produktion von neutralisierenden Antikörpern führen (Vaccine 7, 29 - 33 (1989), Angew. Chem., Int. Ed. 24, 872 - 873 (1989)). Darüber hinaus ist es auch möglich, verschiedene Partialsequenzen kovalent zu verknüpfen und mit einer Membranankerverbindung zu verbinden.

Erfindungsgegenstand ist weiterhin ein Verfahren zur Herstellung einer synthetischen Vakzine, das dadurch gekennzeichnet ist, daß Proteine oder Partialsequenzen von Erregern durch eine Konjugationsreaktion an die Membranankerverbindung gebunden wird. Die Konjugationsreaktion kann z. B. eine Kondensation, Addition, Substitution, Oxidation oder Disulfidbildung sein. Bevorzugte Konjugationsmethoden sind in den Beispielen wiedergegeben. Weitere Konjugationsmethoden sind in der bereits zitierten Deutschen Offenlegungsschrift 35 46 150 beschrieben.

Die Herstellung der Membranankerverbindungen ist ebenfalls in der zuletzt genannten deutschen Offenlegungsschrift ausführlich beschrieben.

Die gegebenenfalls nötige Trennung der Diastereomeren kann nach unterschiedlichen Methoden, wie z. B. in Hoppe-Seyler's Z. Physiolog. Chem. 364 (1983) 593 beschrieben erfolgen.

Der Aufbau der für die Membranankerwirkstoffkonjugate einzusetzenden Partialsequenzen kann auf unterschiedliche, literaturbekannte Weise erfolgen, vgl. z. B. Wünsch et al. in Houben-Weyl, Bd. 15/1.2, Stuttgart, Thieme-Verlag oder Wünsch in Angew. Chem. 83 (1971), E. Gross und J. Meienhofer (Herausgeb.), The Peptides, Vol. 1 (1979), 2 (1979), 3 (1981) und 5 (1983) Academic Press, New York 7713 oder die Deutsche Offenlegungsschrift 35 46 150. In Beispiel 1 wird ein bevorzugtes Verfahren zur Herstellung einer Partialsequenz und eines Konjugats näher erläutert.

Weiterhin gehören zum Erfindungsgegenstand pharmazeutische oder veterinärmedizinische Zubereitungen, die einen Gehalt an Konjugat aus mindestens einer Membranankerverbindung und mindestens einer Partialsequenz eines der genannten Proteine oder Organismen aufweisen. Normalerweise werden zusätzlich neben einem Lösungsmittel keine zusätzlichen Hilfs- und Trägerstoffe oder Adjuvanzien für die erfindungsgemäßen Zubereitungen benötigt. In manchen Fällen kann es aber sinnvoll sein, derartige Hilfs- und/oder Trägerstoffe sowie gegebenenfalls Adjuvanzien den erfindungsgemäßen Zubereitungen zuzusetzen (Anton Mayr, Gerhard Eißnen, Barbara Mayr-Bibrack, Handbuch der Schutzimpfungen in der Tiermedizin, 1984, Verlag Paul Parey, Berlin-Hamburg).

Die Menge an Vakzine, die für eine sichere Immunisierung eines Warmblüters notwendig ist, hängt ab von der Art des Warmblüters, von der bzw. den Membranankerverbindungen und dem Protein oder der bzw. den Partialsequenzen des Organismus, gegen den immunisiert werden soll und ist im Einzelfall empirisch zu ermitteln.

Durch die nachfolgenden Beispiele soll die Erfindung näher erläutert werden.

### Synthese

### Beispiel 1

### Synthese von N-Palmitoyl-S-[2,3-(bispalmitoyloxy)-propyl]cysteinyl-seryl-seryl-NP 147-158

Die Influenza A-Virus-Nukleoprotein-Peptidsequenz wurde durch Festphasenpeptidsynthese synthetisiert. Es wurden Fmoc-Aminosäuren benutzt. Folgende Seitenkettenschutzgruppen kamen zur Anwendung: Thr(tBu), Tyr(tBu), Arg(Pmc). Es wurde 1 g para-Benzyloxybenzylalkohol-Harz, beladen mit 0,5 mmol Fmoc-Gly eingesetzt und die Peptidsequenz nach folgenden Synthesezyklen aufgebaut. N-Aktivierung mit 50 % Piperidin in DMF (1 x 10 min). Kupplung der folgenden Aminosäure für 30 min mit BOP/HOBT [Benzotriazol-1-yl-oxy-tris-(dimethyl amino)-phosphoniumhexafluorophosphat/1-Hydroxybenzo-triazol] und Diisopropylethylamin in DMF. Es wurden jeweils Doppelkupplungen mit 3-fachem Überschuß an Fmoc-Aminosäure und 4,5-fachem Überschuß an Diisopropylethylamin (jeweils bezogen auf freie Aminogruppen am Harz) durchgeführt. Nach jeder Doppelkupplung wurde das Peptidharz je dreimal mit N-Methylpyrrolidon, Dichlormethan und N-Methylpyrrolidon gewaschen.

Nach der Synthese der harzgebundenen Influenza A-Virus-Nukleoprotein-Sequenz wurde ein Teil des Peptids durch Trifluoressigsäurespaltung gewonnen und auf Reinheit geprüft mittels HPLC, MS, Aminosäureanalyse, Analyse auf chiraler Phase sowie Sequenzanalyse. Die HPLC-Prüfung ergab eine Reinheit von über 90 %. Nach Kupplung von zwei Serinresten [Fmoc-Ser(tBu)] an das harzgebundene Peptid, erfolgte die Kupplung des Tripalmitoyl-S-glycerincysteins nach der DIC/HOBT-Methode. Nach vier Stunden wurde ein Äquivalent N-Methylmorpholin hinzugefügt und nach einer weiteren Stunde wurde das Lipopeptid-Harz gewaschen. Das Lipopeptid wurde von 100 mg Harz mittels 2 ml Trifluoressigsäure (mit 100 µl Thioanisol und 100 µg Thiokresol) innerhalb von einer Stunde getrennt. Um die Arg(Pmc)-Schutzgruppen vollständig zu entfernen, wurde zusätzlich 30 min bei 50°C mit Trifluoressigsäure nachbehandelt. Das Filtrat wurde eingedampft, der Rückstand mit Essigsäure aufgenommen und in kalten Ether gegeben. Das ausgefallene Lipopeptid wurde 3 x mit Ether gewaschen und aus tert.-Butanol/Wasser im Verhältnis 3 : 1 lyophilisiert.

### Beispiel 2

### Synthese von N-Palmitoyl-S-[2,3-(bispalmitoyloxy)-propyl]cysteinyl-seryl-seryl-NP (365-380)

Die Synthese erfolgte analog Beispiel 1. Es wurden Fmoc-Aminosäuren mit folgenden Seitenkettenschutzgruppen benutzt:
Ser(tBu), Glu(OtBu), Thr(tBu). Asn wurde ohne Seitenkettenschutzgruppe mittels Diisopropylcarbodiimid/HOBT gekuppelt. Als Ausgangsharz wurde Fmoc-Glu(OtBu)-p-Benzyloxybenzylalkohol-Polystyrol, quervernetzt mit 1 % Divinylbenzol, eingesetzt. Die Beladung an Fmoc-Glu(OtBu) betrug 0,45 mmol/g. Die Abspaltung des Peptids und Pam₃Cys-Ser-Ser-Peptids von je 100 mg Harz erfolgte mit 2 ml Trifluoressigsäure unter Zusatz von 0,1 ml Thioanisol und 100 µg Thiokresol innerhalb von 90 min. Die Sequenz wurde durch Sequenzanalyse des freien Peptids bestätigt; durch HPLC-Prüfung wurde ein einheitlicher Peak mit über 90 % ermittelt. Aminosäurenanalyse und Prüfung auf Enantiomerenreinheit an chiraler Phase ergaben die erwarteten Werte.

### Wirksamkeitstests

### A)

Unter SPF-Bedingungen gezüchtete, 3 Monate alte BALB/c-Inzuchtmäuse wurden intravenös mit 100 µg Pam₃Cys-Ser-Ser-[NP 147-158] immunisiert. (100 µg Pam₃Cys-Ser-Ser-[NP 147-158], aufgenommen in 300 µl PBS, 1 min beschallt). Nach 28 Tagen wurden die Mäuse mit 0,2 bzw. 0,4 haemagglutinierenden Einheiten Influenza-Virus A/PR/8 intranasal infiziert. Analog wurden zur Kontrolle Mäuse infiziert, denen 300 µl PBS intravenös appliziert wurde. Der Verlauf der Infektion wurde anhand von täglichen Gewichtskontrollen und der Überlebensrate kontrolliert. 11 von 12 Kontrolltieren, die mit 0,4 haemagglutinierenden Einheiten infiziert wurden, starben nach 11 Tagen an der Virusinfektion, während von den immunisierten Tieren nur 4 von 12 starben.

Eine weitere Kontrollgruppe und eine mit Pam₃Cys-Ser-Ser-[NP 147-158] immunisierte Gruppe wurden mit 0,2 haemagglutinierenden Einheiten Influenza-Virus infiziert. Nach 18 Tagen lebten noch 40 % der Kontrolltiere (4 von 10 Tieren), während 75 % der immunisierten Tiere lebten. Am Tag 18 betrug die Gewichtsdifferenz zwischen immunisierten Tieren und Kontrolltieren 4 g. Die überlebenden Tiere der Kontrollgruppe verloren weiter an Gewicht, während sich die immunisierten Tiere langsam von der Infektion erholten.

### B)

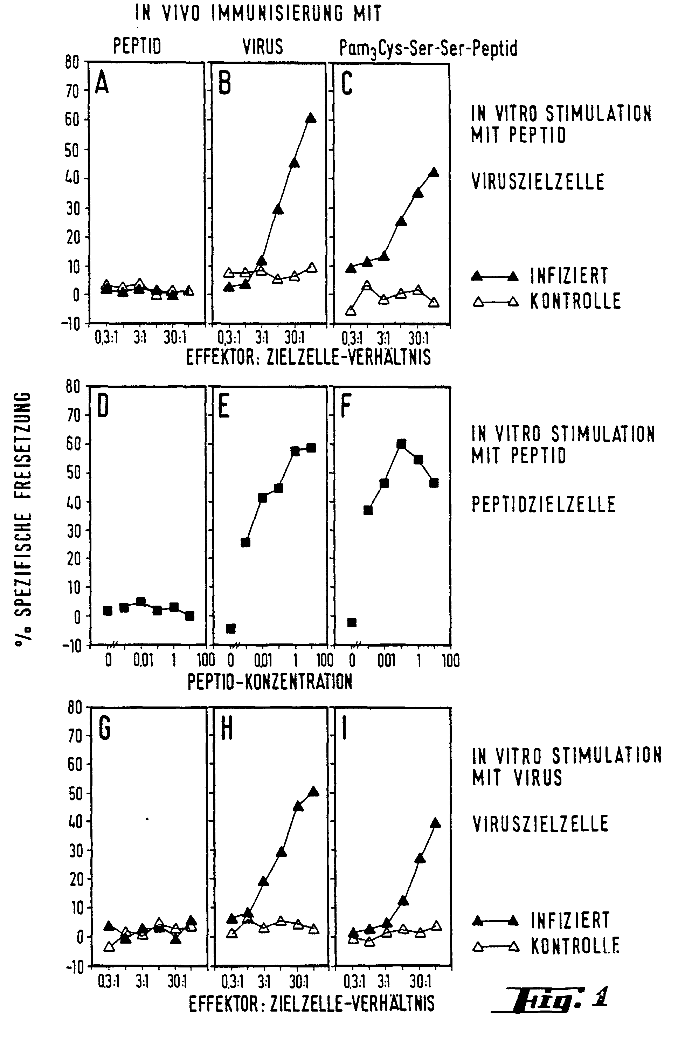

### Zytotoxische T-Zell-Aktivität von Milzzellen aus BALB/c-Mäusen nach Immunisierung mit freiem Peptid, Virus oder Pam₃Cys-Ser-Ser-Peptid (Fig. 1)

BALB/c-Mäuse erhielten durch intravenöse Applikation in 300 µl PBS
a) 8 x 10⁷ mit 1,6 µM Nukleoproteinpeptid 147-158 (R-) vorinkubierte, syngene Milzzellen; (A, D, G),
b) 8 x 10⁷ mit 160 µM Pam₃Cys-Ser-Ser-[NP 147-158 (R-)]-Lipopeptid vorinkubierte, syngene Milzzellen; (C,F),
c) 50 haemagglutinierende Einheiten von Influenza A-Virus PR/8/34; (B,E,H),
d) 100 µg Pam₃Cys-Ser-Ser-[NP 147-158 (R-)]; (I).

Nach 6 Tagen wurden den immunisierten bzw. infizierten Tieren die Milzen entnommen und die Milzzellen 5 Tage lang mit Peptid (A bis F) oder mit Virus PR8 infizierten, syngenen Stimulatorzellen (G,H,I) restimuliert. Dazu wurden je 2,5 x 10⁷-Zellen kultiviert in 10 ml a-MEM-Medium (Hersteller: Gibco), angereichert mit 10 % fötalem Kälberserum, 2-Mercaptoethanol, Glutamin und Antibiotika unter Zusatz von entweder 80 nM NP 147-158(R-)-Peptid (A,F) oder von 5 x 10⁶-Virus PR8-infizierten, mit 20 Gy bestrahlten syngenen Milzzellen (G,H,I). Die Infektion von Stimulator und Zielzellen wurde wie beschrieben durchgeführt (Eur. J. Immunol. 7, 630 - 635 (1977).

Die Aktivität der zytotoxischen T-Zellen wurde durch einen ⁵¹Cr-Release-Standardtest (Eur. J. Immunol. 137, 2.676 - 2.681 (1986)) ermittelt. Schaubilder A, B, C und G, H, I zeigen die CTL-Aktivität auf unbehandelte (△) oder PR8 infizierte (▲) P815 (MHC:H-2^{d})-Zielzellen.

Schaubilder D, E und F zeigen CTL-AKtivität auf P815-Zellen, die mit verschiedenen Konzentrationen von freiem Peptid 30 min bei 37°C vorbehandelt wurden. Hier wurde ein Verhältnis von Effektor zu Zielzelle 30 : 1 eingesetzt.

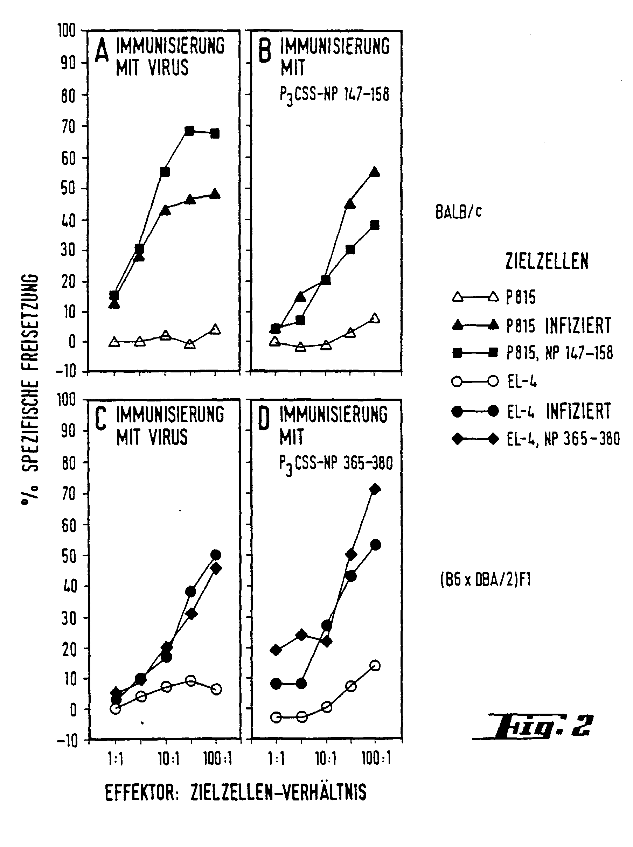

### C) Aktivität zytotoxischer T-Zellen nach Immunisierung von Mäusen mit Pam₃Cys- Ser-Ser-[NP 147-158] oder Pam₃Cys-Ser-Ser-[NP 365 - 380] (Fig. 2)

BALB/c-Mäuse (Schaubilder A,B) oder (B6 x DBA/2)-F1-Mäuse (C,D) wurden mit Influenza A-Virus (A,C) bzw. mit 100 µg Pam₃Cys-Ser-Ser-[NP 147-158] (Bild B) bzw. mit 100 µg Pam₃Cys-Ser-Ser-[NP 365-380] (Bild D) immunisiert. Nach 6 Tagen wurden die Milzen entnommen und die Milzzellen, wie unter B) beschrieben, in Gegenwart von 0,8 µM NP 147-158 Peptid (A,B) bzw. 0,8 µM NP 365-380 Peptid (C,D) stimuliert. Die Aktivität der zytotoxischen T-Zellen wurde anschließend auf unbehandelte P815-Zielzellen (△), auf PR8 infizierte P815-Zielzellen (▲) und auf 90 min bei 37°C mit NP 147-158-Peptid vorinkubierte P815-Zielzellen (■) geprüft; ebenso auf unbehandelte EL-4-(MHC H-2^{d})-Zellen (o), auf PR8 infizierte EL-4-Zielzellen (●), und auf 90 min bei 37°C mit NP 365-380-Peptid vorinkubierte EL-4-Zellen (◆).

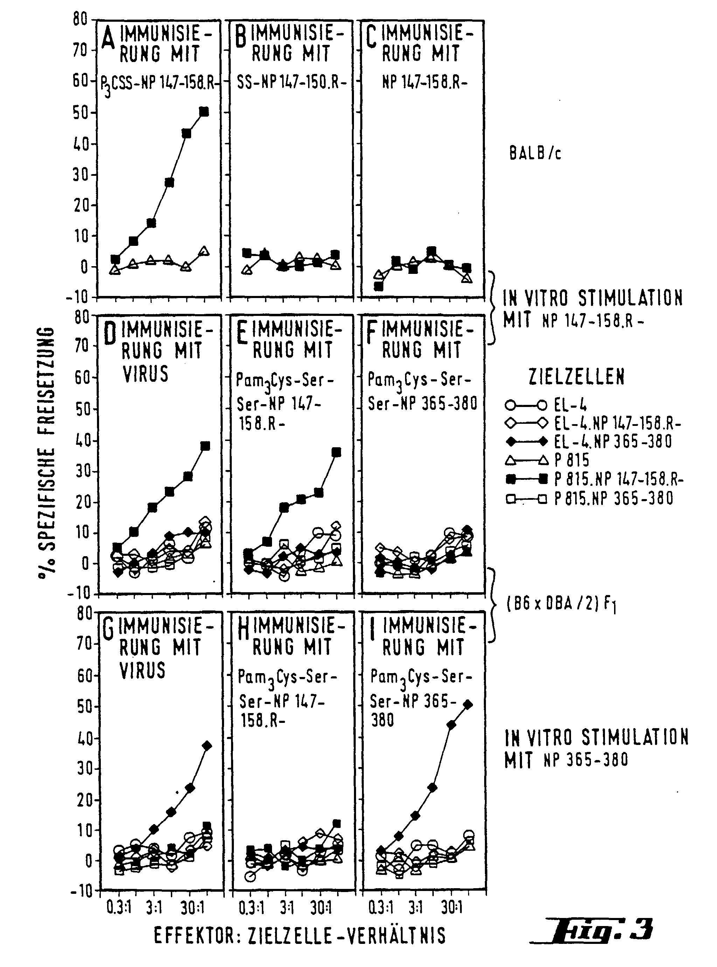

### D) Prüfung auf MHC Klasse I-Restriktion und auf Spezifität der Immunisierung mit Lipopeptid (Fig. 3)

BALB/c-Mäuse (Bilder A,B,C) oder (B6 x DBA/2)-F1-Mäuse (Bilder D-I) erhielten i.v. in 300 µl PBS 100 µg Pam₃Cys-Ser-Ser-[NP 147-158(R-)] (Bilder A,E,H) bzw.
50 µg Ser-Ser-[NP 147-158(R-)] (Bild B) bzw.
50 µg [NP 147-158(R-)] (Bild C) bzw.
50 haemagglutinierenden Einheiten Influenza PR8-Virus (Bilder D,G) bzw. 100 µg Pam₃Cys-Ser-Ser-[NP 365-380] (Bilder F,I).

Sechs Tage nach der Injektion wurden die Milzzellen, wie in Beispiel 2 beschrieben, kultiviert unter Zusatz von Nukleoprotein 147-158(R-)-Peptid (Bilder A - F), oder Nukleoprotein 365-380-Peptid (Bilder G - I).
Die Aktivität der erhaltenen cytotoxischen T-Zellen wurde bestimmt gegen
- unbehandelte P815-Zielzellen (△)
- 90 min bei 37°C mit NP 147-158(R-) vorinkubierte P815-Zielzellen (■)
- 90 min bei 37°C mit NP 365-380 vorinkubierte P815-Zielzellen (□)
- unbehandelte EL-4-Zielzellen (○)
- 90 min bei 37°C mit NP 147-158(R-) vorinkubierte EL-4-Zielzellen (◇)
- 90 min bei 37°C mit NP 365-380 vorinkubierte EL-4-Zielzellen (◆).

## Patentansprüche (Patentansprüche für folgende(n) Vertragsstaat(en): AT, BE, CH, DE, DK, FR, GB, IT, LI, LU, NL, SE)

1. Synthetische Vakzine zur spezifischen Induktion von zytotoxischen T-Lymphozyten, bestehend aus einem Konjugat aus mindestens einer Membranankerverbindung und einem mindestens ein Killer-T-Zellepitop enthaltenden Protein eines Virus, eines Bakteriums, eines Parasiten oder eines Tumorantigens oder mindestens einer mindestens ein Killer-T-Zellepitop enthaltenden Partialsequenz eines Virus-, Bakterium- oder Parasiten-Proteins oder eines Tumorantigens wobei die Partialsequenzen des Maul und Klauenseuche Virus ausgenommen sind, dadurch gekennzeichnet, daß die Membranankerverbindung eine der nachstehenden Formeln aufweist 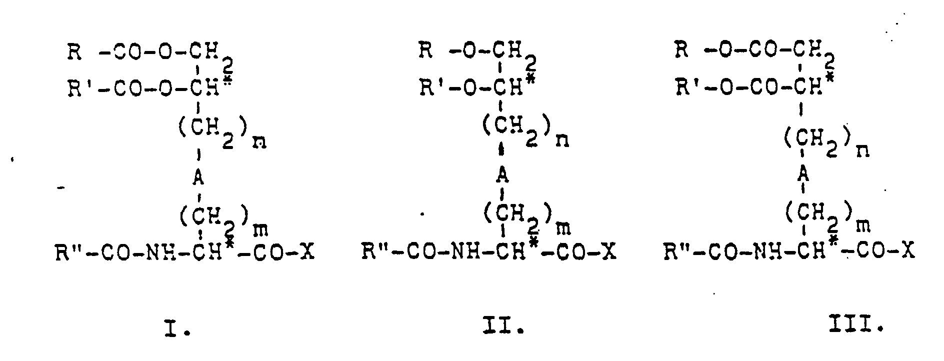 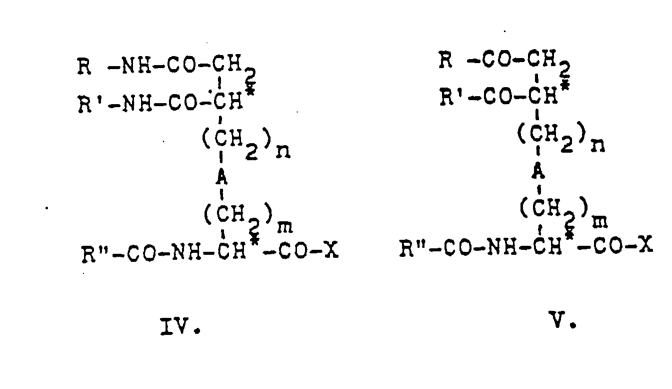 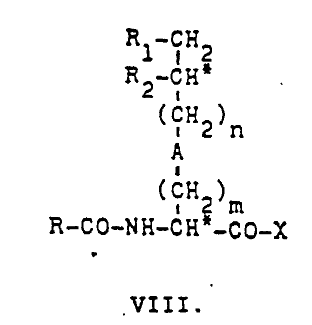 in denen
A Schwefel, Sauerstoff, Disulfid (-S-S-), Methylen (-CH₂-) oder -NH- sein kann;
n = 0 bis 5, m = 1 oder 2;
C^{*} ein asymmetrisches Kohlenstoffatom mit R- oder S-Konfiguration ist,
R, R' und R" gleich oder verschieden sind und eine Alkyl-, Alkenyl- oder Alkinylgruppe mit 7 bis 25 Kohlenstoffatomen sind, welche mit Hydroxy-, Amino-, Oxo-, Acyl-, Alkyl- oder Cycloalkylgruppen substituiert sein kann, und R₁ und R₂ gleich oder verschieden sind und dieselben Bedeutungen wie R, R' und R" haben, aber auch -OR, -O-COR, -COOR, -NHCOR oder -CONHR sein können, wobei X eine Kette von bis zu 10 Aminosäuren ist, an die das Protein oder die Partialsequenz des Virus-, Bakterium- oder Parasiten-Proteins oder eines Tumorantigens gebunden ist, oder das Protein oder die Partialsequenz selbst ist.

2. Synthetische Vakzine gemäß Anspruch 1, dadurch gekennzeichnet, daß die Membranankerverbindung N-Palmitoyl-S- 2,3-(bispalmitoyloxy)-propyl-cysteinyl-seryl-serin ist, wobei die Partialsequenz an den terminalen Serinrest gebunden ist.

3. Synthetische Vakzine gemäß den Ansprüche 1 oder 2, dadurch gekennzeichnet, daß das Protein oder die Partialsequenz von einem Adenovirus, HIV, Influenza-Virus, LCMV, MCMV, Hepatitis-Virus, HTLV, FELV, Treponema pallidum, Gonokokkus, Bordetella pertussis oder Plasmodium spec. oder einem anderen ein Killer-T-Zell-Epitop enthaltenden Pathogen stammt.

4. Synthetische Vakzine gemäß einem oder mehreren der Ansprüche 1 -3, dadurch gekennzeichnet, daß ein Gemisch aus Membranankerwirkstoffkonjugaten mit verschiedenen Partialsequenzen vorliegt.

5. Synthetische Vakzine gemäß Anspruch 1, dadurch gekennzeichnet, daß neben Membranankerwirkstoffkonjugaten zur Induktion zytotoxischer T-Lymphozyten auch Membranankerwirkstoffkonjugate zur Erzeugung neutralisierender Antikörper vorhanden sind.

6. Verfahren zur Herstellung einer synthetischen Vakzine gemäß einem oder mehreren der Ansprüche 1 - 5, dadurch gekennzeichnet, daß ein Membranankerwirkstoffkonjugat nach bekannten Methoden synthetisiert wird.

7. Pharmazeutische oder veterinärmedizische Zubereitung zur Induktion von zytotoxischen T-Lymphozyten, gekennzeichnet durch einen Gehalt an einer synthetischen Vakzine gemäß einem oder mehreren der Ansprüche 1 -5, gegebenenfalls neben üblichen Hilfs- und/oder Trägerstoffen und gegebenenfalls neben weiteren Vakzinen.

## Patentansprüche (Patentansprüche für folgende(n) Vertragsstaat(en): ES, GR)

1. Verfahren zum Herstellen einer synthetischen Vakzine zur spezifischen Induktion von zytotoxischen T-Lymphozyten, die aus einem Konjugat aus mindestens einer Membranankerverbindung und einem mindestens ein Killer-T-Zellepitop enthaltenden Protein eines Virus, eines Bakteriums, eines Parasiten oder eines Tumorantigens oder mindestens einer mindestens ein Killer-T-Zellepitop enthaltenden Partialsequenz eines Virus-, Bakterium- oder Parasiten-Proteins oder eines Tumorantigens besteht, dadurch gekennzeichnet, daß ein Membranankerwirkstoffkonjugat nach bekannten Methoden synthetisiert wird, wobei die Partialsequenzen des Maul und Klauenseuche ausgenommen sind und wobei die Membranankerverbindung eine der nachstehenden Formeln aufweist 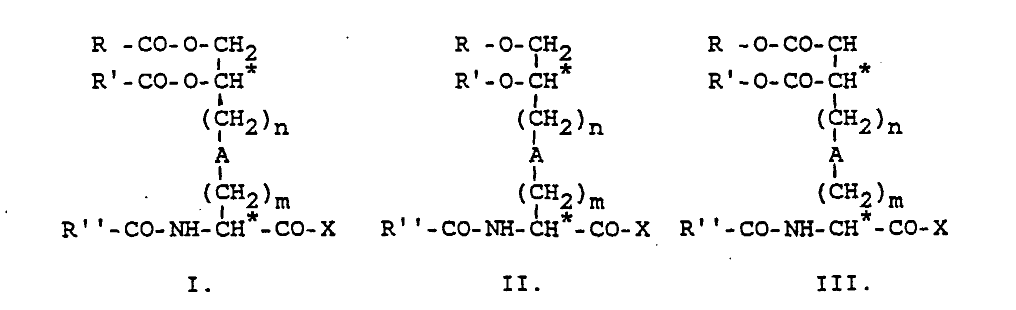 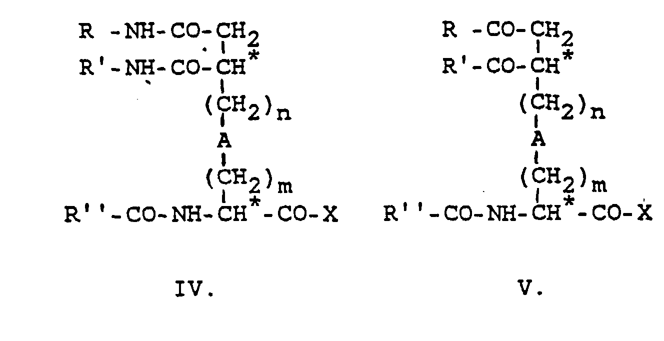 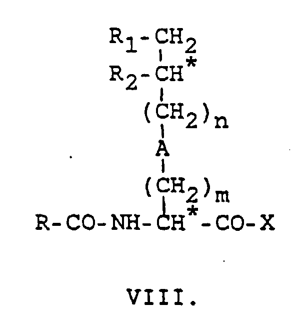 in denen
A Schwefel, Sauerstoff, Disulfid (-S-S-), Methylen (-CH₂-) oder -NH- sein kann;
n = 0 bis 5, m = 1 oder 2;
C^{*} ein asymmetrisches Kohlenstoffatom mit R- oder S-Konfiguration ist,
R, R' und R" gleich oder verschieden sind und Wasserstoff oder eine Alkyl-, Alkenyl- oder Alkinylgruppe mit 7 bis 25 Kohlenstoffatomen sind, welche mit Hydroxy-, Amino-, Oxo-, Acyl-, Alkyl- oder Cycloalkylgruppen substituiert sein kann, und.
R₁ und R₂ gleich oder verschieden sind und dieselben Bedeutungen wie R, R' und R" haben, aber auch -OR, -O-COR, -COOR, -NHCOR oder -CONHR sein können, wobei X eine Kette von bis zu 10 Aminosäuren ist, an die das Protein oder die Partialsequenz des Virus-, Bakterium- oder Parasiten-Proteins oder eines Tumorantigens gebunden ist, oder das Protein oder die Partialsequenz selbst ist.

2. Verfahren nach Anspruch 1, dadurch gekennzeichnet, daß die Membranankerverbindung N-Palmitoyl-S-2,3- (bispalmitoyloxy)-propyl- cysteinyl-seryl-serin ist, wobei die Partialsequenz an den terminalen Serinrest gebunden ist.

3. Verfahren nach Anspruch 1, dadurch gekennzeichnet, daß das Protein oder die Partialsequenz von einem Adenovirus, HIV, Influenza-Virus, LCMV, MCMV, Hepatitis-Virus, HTLV, FELV, Treponema pallidum, Gonokokkus, Bordetella pertussis oder Plasmodium spec. oder einem anderen ein Killer-T-Zell-Epitop enthaltenden Pathogen stammt.

4. Verfahren nach Anspruch 1, dadurch gekennzeichnet, daß ein Gemisch aus Membranankerwirkstoffkonjugaten mit verschiedenen Partialsequenzen hergestellt wird.

5. Verfahren nach Anspruch 1, dadurch gekennzeichnet, daß eine Vakzine hergestellt wird, in der neben Membranankerwirkstoffkonjugaten zur Induktion zytotoxischer T-Lymphozyten auch Membranankerwirkstoffkonjugate zur Erzeugung neutralisierender Antikörper vorhanden sind.

## Claims (Claims for the following Contracting State(s): AT, BE, CH, DE, DK, FR, GB, IT, LI, LU, NL, SE)

1. A synthetic vaccine for the specific induction of cytotoxic T-lymphocytes, comprising a conjugate of at least one membrane anchor compound and a protein, containing at least one killer T-cell epitope, of a virus, of a bacterium, of a parasite or of a tumor antigen or at least one partial sequence, containing at least one killer T-cell epitope, of a viral, bacterial or parasite protein or of a tumor antigen, with the exception of the partial sequences of the foot-and-mouth disease virus, wherein the membrane anchor compound has one of the formulae below 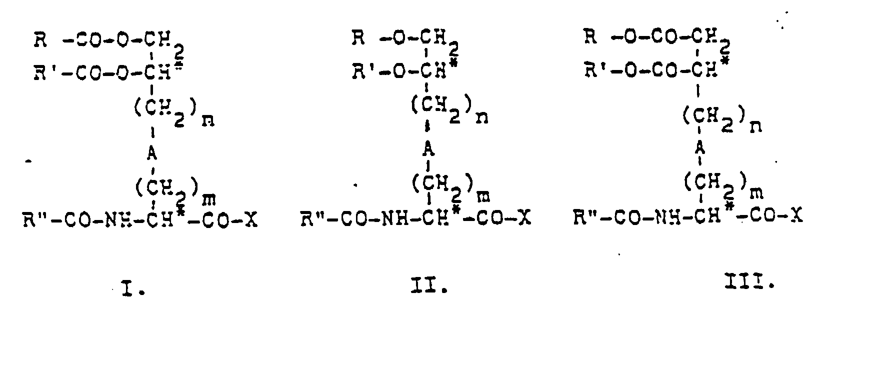 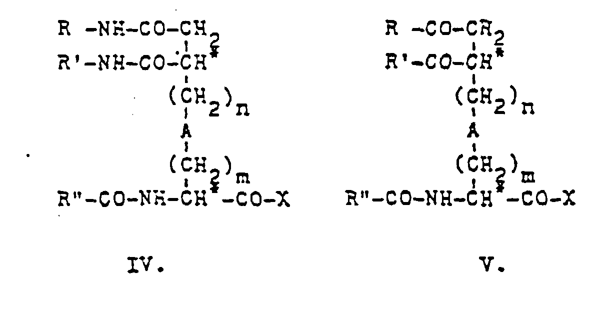 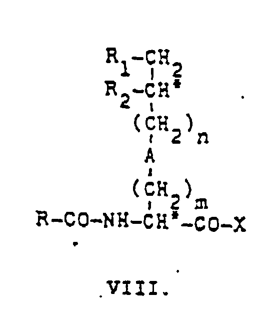 in which
A can be sulfur, oxygen, disulfide (-S-S-), methylene (-CH₂-) or -NH-;
n = 0 to 5, m = 1 or 2;
C^{*} is an asymmetric carbon atom with an R or S configuration,
R, R' and R" are identical or different and are an alkyl, alkenyl or alkynyl group having 7 to 25 carbon atoms, which can be substituted by hydroxyl, amino, oxo, acyl, alkyl or cycloalkyl groups, and R₁ and R₂ are identical or different and have the same meanings as R, R' and R" but can also be -OR, -O-COR, -COOR, -NHCOR or -CONHR, where X is a chain of up to 10 amino acids to which the protein or the partial sequence of the viral, bacterial or parasite protein or of a tumor antigen is bonded, or is the protein or the partial sequence itself.

2. The synthetic vaccine as claimed in claim 1, wherein the membrane anchor compound is N-palmitoyl -S-2,3 - (bispalmitoyloxy) -propyl-cysteinyl-seryl-serine, the partial sequence being bonded to the terminal serine residue.

3. The synthetic vaccine as claimed in claims 1 or 2, wherein the protein or the partial sequence is derived from an adenovirus, HIV, influenza virus, LCMV, MCMV, hepatitis virus, HTLV, FELV, Treponema pallidum, gonococcus, Bordetella pertussis or Plasmodium spec. or another pathogen containing a killer T-cell epitope.

4. The synthetic vaccine as claimed in one or more of claims 1 - 3, wherein a mixture of membrane anchor/active compound conjugates with various partial sequences is present.

5. The synthetic vaccine as claimed in claim 1, wherein, in addition to membrane anchor/active compound conjugates for the induction of cytotoxic T-lymphocytes, membrane anchor/active compound conjugates for the generation of neutralizing antibodies are also present.

6. A process for the preparation of a synthetic vaccine as claimed in one or more of claims 1 - 5, which comprises synthesizing a membrane anchor/active compound conjugate by known methods.

7. A pharmaceutical preparation or preparation for veterinary medicine for the induction of cytotoxic T-lymphocytes, which contains a synthetic vaccine as claimed in one or more of claims 1 - 5, if desired in addition to customary auxiliaries and/or excipients and, if desired, in addition to further vaccines.

## Claims (Claims for the following Contracting State(s): ES, GR)

1. A process for preparing a synthetic vaccine for the specific induction of cytotoxic T-lymphocytes, comprising a conjugate of at least one membrane anchor compound and a protein, containing at least one killer T-cell epitope, of a virus, of a bacterium, of a parasite or of a tumor antigen or at least one partial sequence, containing at least one killer T-cell epitope, of a viral, bacterial or parasite protein or of a tumor antigen, which comprises synthesizing a membrane anchor/active compound conjugate by known methods, with the exception of the partial sequences of the foot-and-mouth disease virus, and the membrane anchor compound having one of the formulae below 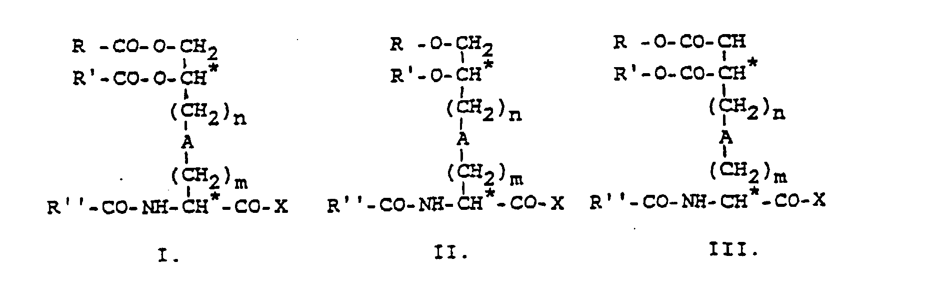 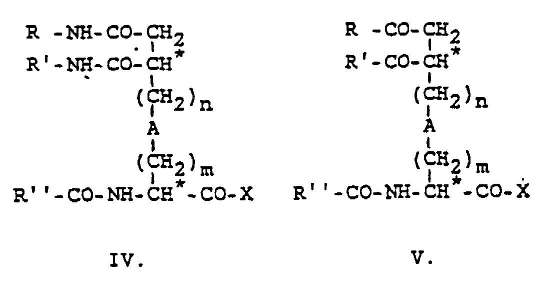 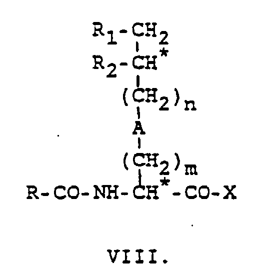 in which
A can be sulfur, oxygen, disulfide (-S-S-), methylene (-CH₂-) or -NH-;
n = 0 to 5, m = 1 or 2;
C^{*} is an asymmetric carbon atom with an R or S configuration,
R, R' and R" are identical or different and are hydrogen or an alkyl, alkenyl or alkynyl group having 7 to 25 carbon atoms, which can be substituted by hydroxyl, amino, oxo, acyl, alkyl or cycloalkyl groups, and R₁ and R₂ are identical or different and have the same meanings as R, R' and R" but can also be -OR, -O-COR, -COOR, -NHCOR or -CONHR, where X is a chain of up to 10 amino acids to which the protein or the partial sequence of the viral, bacterial or parasite protein or of a tumor antigen is bonded, or is the protein or the partial sequence itself.

2. The process as claimed in claim 1, wherein the membrane anchor compound is N-palmitoyl-S-2,3-(bispalmitoyloxy) -propyl-cysteinyl-seryl-serine, the partial sequence being bonded to the terminal serine residue.

3. The process as claimed in claim 1, wherein the protein or the partial sequence is derived from an adenovirus, HIV, influenza virus, LCMV, MCMV, hepatitis virus, HTLV, FELV, Treponema pallidum, gonococcus, Bordetella pertussis or Plasmodium spec. or another pathogen containing a killer T-cell epitope.

4. The process as claimed in claim 1, wherein a mixture of membrane anchor/active compound conjugates with various partial sequences is produced.

5. The process as claimed in claim 1, wherein there is preparation of a vaccine in which, in addition to membrane anchor/active compound conjugates for the induction of cytotoxic T-lymphocytes, membrane anchor/active compound conjugates for the generation of neutralizing antibodies are also present.

## Revendications (Revendications pour l'(les) Etat(s) contractant(s) suivant(s): AT, BE, CH, DE, DK, FR, GB, IT, LI, LU, NL, SE)

1. Vaccin synthétique pour l'induction spécifique des lymphocytes-T cytotoxiques constitué d'un conjugué dérivé d'au moins un composé d'ancrage à la membrane et d'une protéine d'un virus, d'une bactérie, d'un parasite ou d'un antigène tumoral contenant au moins un épitope de cellule T tueuse ou d'au moins une séquence partielle d'une protéine d'un virus, d'une bactérie ou d'un parasite contenant au moins un épitope de cellule T tueuse ou d'un antigène tumoral, les séquences partielles du virus de la fièvre aphteuse étant exclues, ledit vaccin étant caractérisé en ce que le composé d'ancrage à la membrane a l'une des formules suivantes : 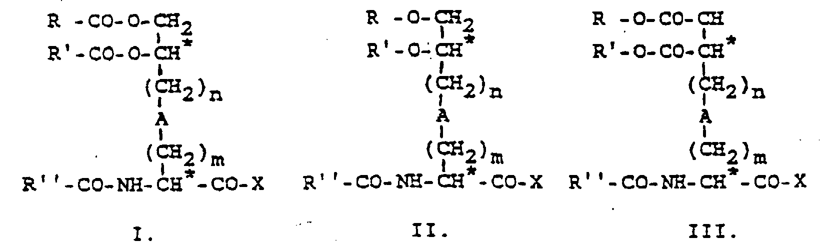 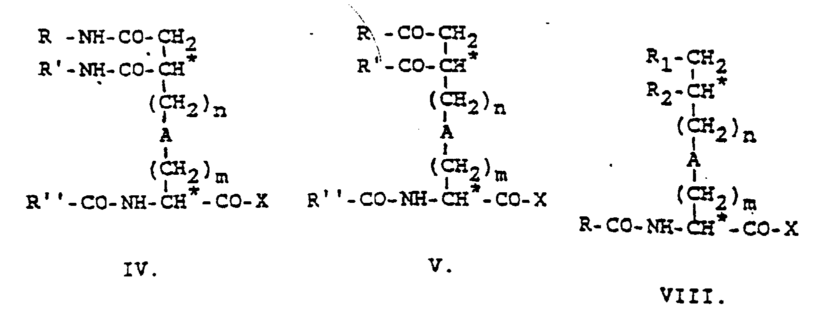 dans lesquelles
A peut représenter un atome de soufre, un atome d'oxygène, un pont disulfure (-SS-), un groupe méthylène (-CH₂-) ou -NH-;
n = 0 à 5, m =1 ou 2 ;
C^{*} représente un atome de carbone asymétrique de configuration R ou S ;
R, R' et R" sont identiques ou différents et représentent un groupe alkyle, alcényle ou alcynyle de 7 à 25 atomes de carbone, lesquels peuvent être substitués par des groupes hydroxy, amino, oxo, acyle, alkyle ou cycloalkyle et R₁ et R₂ sont identiques ou différents et ont les mêmes significations que R, R'et R", mais peuvent également représenter-OR, -O-COR, -COOR, -NHCOR ou -CONHR, où X est une chaîne d'au plus 10 aminoacides, à laquelle est liée la protéine ou la séquence partielle de la protéine d'un virus, d'une bactérie ou d'un parasite ou d'un antigène tumoral, ou bien X est elle-même la protéine ou la séquence partielle.

2. Vaccin synthétique selon la revendication 1, caractérisé en ce que le composé d'ancrage à la membrane est la N-palmitoyl -S-2,3-(bispalmitoyloxy)-propyl-cystéinyl-séryl-sérine, où la séquence partielle est liée au résidu sérine terminal.

3. Vaccin synthétique selon l'une ou plusieurs des revendications 1-2, caractérisé en ce que la protéine ou la séquence partielle dérive d'un adénovirus, de l'HIV, du virus de la grippe, de LCMV, de MCMV, du virus de l'hépatite, de HTLV, de FELV, de *Treponema pallidum,* de *Gonococcus,* de *Bordetella pertussis* ou de *Plasmodium sp.* ou d'un autre agent pathogène contenant un épitope de cellule T tueuse.

4. Vaccin synthétique selon l'une ou plusieurs des revendications 1-3, caractérisé en ce qu'il présente un mélange de conjugués de substance active d'ancrage à la membrane avec diverses séquences partielles.

5. Vaccin synthétique selon la revendication 1, caractérisé en ce qu'au côté des conjugués de substance active d'ancrage à la membrane pour l'induction de lymphocytes-T cytotoxiques sont également présents des conjugués de substance active d'ancrage à la membrane pour la fabrication d'anticorps à action neutralisante.

6. Procédé pour la préparation d'un vaccin synthétique selon l'une ou plusieurs des revendications 1 à 5, caractérisé en ce que l'on synthétise un conjugué de substance active d'ancrage à la membrane selon des méthodes connues.

7. Composition vétérinaire ou pharmaceutique pour l'induction de lymphocytes-T cytotoxiques, caractérisée en ce qu'elle comprend un vaccin synthétique selon l'une ou plusieurs des revendications 1-5, éventuellement au côté d'additifs et/ou de véhicules et éventuellement d'autres vaccins.

## Revendications (Revendications pour l'(les) Etat(s) contractant(s) suivant(s): ES, GR)

1. Procédé pour la préparation d'un vaccin synthétique pour l'induction spécifique des lymphocytes-T cytotoxiques, qui sont constitués d'un conjugué dérivé d'au moins un composé d'ancrage à la membrane et d'une protéine d'un virus, d'une bactérie, d'un parasite ou d'un antigène tumoral contenant au moins un épitope de cellule T tueuse ou d'au moins une séquence partielle d'une protéine d'un virus, d'une bactérie ou d'un parasite contenant au moins un épitope de cellule T tueuse ou d'un antigène tumoral, caractérisé en ce que l'on synthétise un conjugué de substance active d'ancrage à la membrane selon des méthodes connues, les séquences partielles du virus de la fièvre aphteuse étant exclues, dans lequel le composé d'ancrage à la membrane a l'une des formules suivantes : 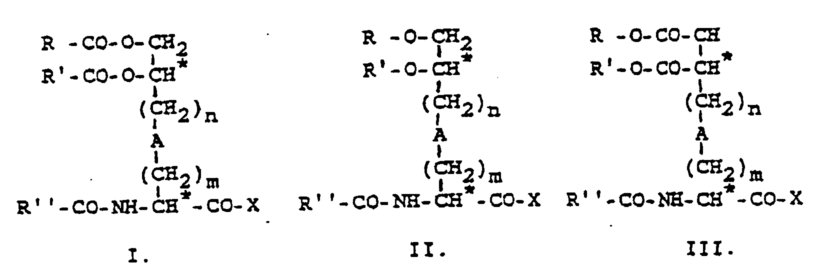 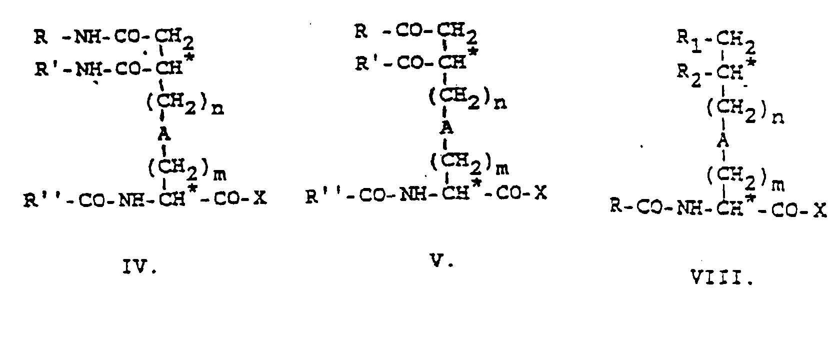 dans lesquelles
A peut représenter un atome de soufre, un atome d'oxygène, un pont disulfure (-SS-), un groupe méthylène (-CH₂-) ou -NH-;
n = 0 à 5, m = 1 ou 2;
C^{*} représente un atome de carbone asymétrique de configuration R ou S ;
R, R' et R" sont identiques ou différents et représentent un atome d'hydrogène ou un groupe alkyle, alcényle ou alcynyle de 7 à 25 atomes de carbone, lesquels peuvent être substitués par des groupes hydroxy, amino, oxo, acyle, alkyle ou cycloalkyle et R₁ et R₂ sont identiques ou différents et ont les mêmes significations que R, R' et R", mais peuvent également représenter-OR, -O-COR, -COOR, -NHCOR ou -CONHR, où X est une chaîne d'au plus 10 aminoacides, à laquelle est liée la protéine ou la séquence partielle de la protéine d'un virus, d'une bactérie ou d'un parasite ou d'un antigène tumoral, ou bien X est elle-même la protéine ou la séquence partielle.

2. Procédé selon la revendication 1, caractérisé en ce que le composé d'ancrage à la membrane est la N-palmitoyl-S-2,3-(bispalmitoyloxy)-propyl-cystéinyl-séryl-sérine, où la séquence partielle est liée au résidu sérine terminal.

3. Procédé selon la revendication 1, caractérisé en ce que la protéine ou la séquence partielle dérivé d'un adénovirus, de l'HIV, du virus de la grippe, de LCMV, de MCMV, du virus de l'hépatite, de HTLV, de FELV, de *Treponema pallidum,* de *Gonococcus,* de *Bordella pertussis* ou de *Plasmodium sp.* ou d'un autre agent pathogène contenant un épitope de cellule T tueuse.

4. Procédé selon la revendication 1, caractérisé en ce que l'on prépare un mélange de conjugués de substance active d'ancrage à la membrane avec diverses séquences partielles.

5. Procédé selon la revendication 1, caractérisé en ce que l'on prépare un vaccin dans lequel, au côté des conjugués de substance active d'ancrage à la membrane pour l'induction de lymphocytes-T cytotoxiques, sont également présents des conjugués de substance active d'ancrage à la membrane pour la fabrication d'anticorps à action neutralisante.
